# EUROPEAN PATENT SPECIFICATION

(11) **EP 2 942 010 B1**
(45) Date of publication and mention of the grant of the patent: **19.07.2017**
(21) Application number: 13883101.1
(22) Date of filing: 26.06.2013
(51) Int. Cl.: A61B 5/12, A61M 21/00, H04R 25/00

(54) **TINNITUS DIAGNOSIS AND TEST DEVICE**
TINNITUSDIAGNOSE- UND -TESTVORRICHTUNG
DISPOSITIF DE TEST ET DE DIAGNOSTIC D'ACOUPHÈNES

(30) Priority: 27.04.2013 CN 201310152231
(43) Date of publication of application: 11.11.2015
(73) Proprietor: Jiangsu Betterlife Medical Co., Ltd., Jiangsu 215500 (CN)
(72) Inventor: ZHAO, Bingbing, Jiangsu 215500 (CN); ZHAO, Yong David, Jiangsu 215500 (CN); ZHAO, Jennifer Jinping, Jiangsu 215500 (CN)
(74) Representative: Becker Kurig Straus
(86) International application number: PCT/CN2013/000761
(87) International publication number: WO 2014/172814

(56) References cited:
- WO-A1-2008/083429
- WO-A2-2008/011396
- WO-A2-2009/002539
- CN-A- 102 647 944
- CN-A- 103 494 669
- CN-U- 203 244 395
- CN-Y- 201 139 570
- US-A1- 2011 295 166
- US-A1- 2012 023 130
- US-A1- 2012 203 130
- US-A1- 2012 283 593

## Description

### FIELD OF THE INVENTION

The present invention relates to the field of medical equipment, particularly a tinnitus diagnosing and testing device.

### BACKGROUND OF THE INVENTION

Tinnitus is a common clinical symptom, usually the subjective perception of sound in ears or in the head without any corresponding external sound source or electromagnetic stimulation, i.e. subjective tinnitus, referred to as "tinnitus" or "cranial tinnitus" for short. In broad terms, tinnitus further contains objective tinnitus in the presence of corresponding sound source, such as vasculogenic or myogenic noise. Tinnitus is different from acousma, which is the perception of sound or music of specific content heard by patients in the absence of external sound source.

It would be difficult to confirm the causes of most tinnitus. Currently, there is no medicine for curing tinnitus approved by SPDA. For most unexplained tinnitus patients, some tinnitus treatment can be provided to relieve the tinnitus or the effect of tinnitus on sufferers. Tinnitus treatment prescription mainly includes two physical methods: tinnitus masking and tinnitus retraining, which are based on retraining or recoding the neutral system to reduce the central excitability, increase the central suppression, cut the vicious cycle of tinnitus and bad mood, so that the patients can accommodate tinnitus, do not perceive or reduce the perception of tinnitus, so as to achieve the purpose of treatment. The sound of tinnitus in clinical practice is infinite, varies from person to person, or varies from left ear to right ear, or even from different time or environment. The key of tinnitus physical therapy is the accurate detection of personalized tinnitus.

However, existing testing equipment is incapable of accurately restoring or diagnosing the audio and tone information of protean tinnitus via few simple pure tone or noise stimulation. Therefore, it is impossible to provide a personalized and purposeful treatment prescription for a patient, subsequently the treatment effect is reduced greatly.

### SUMMARY OF THE INVENTION

The main object of the present invention is to provide a tinnitus diagnosing and testing device, which could accurately restore or diagnose the audio and tone information of tinnitus and provide all kinds of tones, melodies, rhythms and loudness required for a tinnitus diagnosis.

To solve the above problem, one solution of the present invention is to provide a tinnitus diagnosing and testing according to claim 1. Further described are an all-digital audio and tone signal synthesis generators, generating audio and tone signals of all kinds of tones, melodies, rhythms and loudness required for diagnosing a variety of tinnitus mode and/or tinnitus sound;
a control module, controlling said all-digital audio and tone signal synthesis generators for the left ear and right ear respectively and/or interactive simultaneously to comprehensively assess tinnitus and output audio and tone signal of the tinnitus diagnosis. The output audio and tone signal of the tinnitus diagnosis includes pure tone, pulse pure tone, mixed pure tone, mixed pulse pure tone; white noise, colored noise, wideband and narrowband noise; single warble tone, mixed warble tone, pulse warble tone; recording audio signal; preset various tinnitus sound and nature sound module; the analysis and masking of signal data such as Fredman masking curve. masking audiogram, the upper band and the lower band of the masking thresholds, the identification and output of the retraining therapy prescription.

Said all-digital audio and tone signal synthesis generators includes a composite sound signal generating module, a pure tone signal generating module, a noise signal generating module and a record signal generating module. Said composite sound signal generating module, the pure tone signal generating module, the noise signal generating module and the record signal generating module generate specific audio and tone signals respectively. Further, said tinnitus diagnosing and testing device includes:
an all-digital audio and tone signal synthesis generator for the left ear, generating the audio and tone signal required for the left ear;
an all-digital audio and tone signal synthesis generator for the right ear, generating the audio and tone signal required for the right ear;
said control module, interactively controlling said all-digital audio and tone signal synthesis generator for the left ear and said all-digital audio and tone signal synthesis generator for the right ear respectively and/or simultaneously, optimal regulating the audio and tone signal input into the left and right ears, steadily restoring the tinnitus sound of the left and right ears, comprehensive assessing tinnitus and outputting the audio and tone signal of tinnitus diagnosis. The output audio and tone signal of the tinnitus diagnosis includes pure tone, pulse pure tone, mixed pure tone, mixed pulse pure tone; white noise, colored noise, wideband and narrowband noise; single warble tone, mixed warble tone, pulse warble tone; recording audio signal; preset various tinnitus sound and nature sound module; the analysis and masking of signal data such as Fredman masking curve, masking audiogram, the upper band and the lower band of the masking thresholds, the identification and output of the retraining therapy prescription. Further, the data of said all-digital audio and tone signal synthesis generator for the left ear and said all-digital audio and tone signal synthesis generator for the right ear can replicate with each other and be adjusted based on the replication. Futher, said composite sound signal generating module includes:
   a multi-track synthesis module, comprising one or more separate single track signal generator. Each single track signal generator makes sounds at any setting ranges of frequency and acoustic pressure level, and each sound track's contribution to the composite sound depends on the loudness at the given frequency. The tone, melody, rhythm and loudness of composite sound audio and tone signal depend on the arrangement of multiple mono-track signal generators and the contribution of each mono-track signal generator. The overall loudness of the composite sound can be adjusted individually;
   a preset signal waveform module, adjusting the waveform parameters according to the preset audio signal waveform, which includes but not limited to straight wave, shock wave, ramp wave, sawtooth wave, trigonometric function wave (sine wave, cosine wave, tangent wave, cotangent wave), trapezoidal wave, rectangular wave and impulse wave;
   a tinnitus signal library module, saving and calling the tinnitus digital signals, and classified saving and calling all kinds of tinnitus digital signals. According to the personal testing demands of tinnitus patients, it can output a tinnitus sound signal meeting the patients' personalizing diagnosis requirements by fine-tuning and calling the parameters corresponding to tinnitus reference signals, such as frequency, loudness, waveform, etc. Further, said parameters of waveform include, but not limited to, waveform's slope, waveform's amplitude, the prolongation of waveform's peak value, the prolongation of waveform's valley value, wave period and waveform duration. Further, the pure tone signals generated by said pure tone signal generating module include, but not limited to, normal pure tone, synthesis of pure tone, pulse pure tone and mixed pulse pure tone. Further, the noises generated by the noise signal generating module include, but not limited to, white noise, colored noise transformed through a filter, and all kinds of noises with adjustable frequency band-width, tone and loudness. Furthermore, said all-digital audio and tone signal synthesis generator has an adjustable range of acoustic pressure level of 0db-120db in broadband range (such as 30KHz). Furthermore, said tinnitus diagnosing and testing device further comprises a remote control module, which can realize the remote control and information transmission. Furthermore, said tinnitus diagnosing and testing device further comprises an audio signal calibrating module, which can scan and calibrate the audio signal manually or automatically.

The present invention includes the beneficial effects as below. The tinnitus diagnosing and testing device of present invention can accurately restore or simulate each piece of audio and tone information of tinnitus observed in clinical practice, varying from person to person, from left ear to right ear, or from different time or environment. It can test the left and right ears respectively and/or simultaneously, optimized regulate the audio and tone signals input into the left and right ears, steadily restore the tinnitus sound of left and right ears, comprehensive assess tinnitus and output the audio and tone signals of tinnitus diagnosis, then save the result respectively, which establishes a good foundation for patients' personalized treatment prescription, subsequently achieving a better effect of tinnitus remedy. The output audio and tone signal of the tinnitus diagnosis includes pure tone, pulse pure tone, mixed pure tone, mixed pulse pure tone; white noise, colored noise, wideband and narrowband noise; single warble tone, mixed warble tone, pulse warble tone; recording audio signal; preset various tinnitus sound and nature sound module; the analysis and masking of signal data such as Fredman masking curve, masking audiogram, the upper band and the lower band of the masking thresholds, the identification and output of the retraining therapy prescription.

### BRIEF DESCRIPTION OF THE DRAWINGS

Fig.1 is a structure diagram showing a tinnitus diagnosing and testing device in accordance with a preferred embodiment of the invention.
Fig.2 is a structure diagram of an all-digital audio and tone signal synthesis generator in Fig. 1.

### DETAILED DESCRIPTION OF THE INVENTION

Preferred embodiments of the invention will now be described in detail with reference to the drawings, thereby the advantage and virtue of the present invention will become more easily understood by a person skilled in the art, and the scope of the present invention can be defined more clearly.

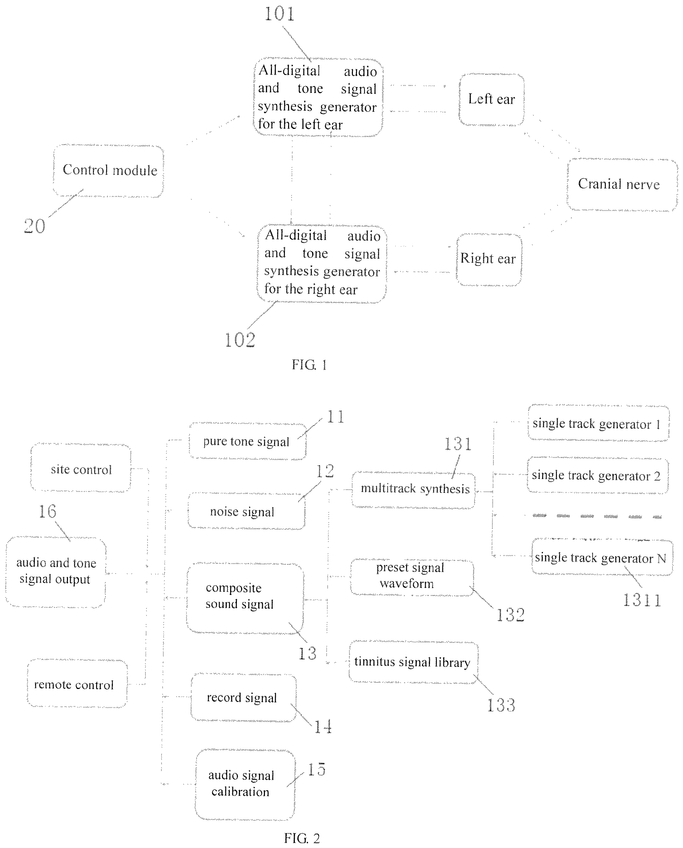

Referring to FIG.1 and FIG.2, the embodiments of the present invention include the followings:
A tinnitus diagnosing and testing device includes: an all-digital audio and tone signal synthesis generator for the left ear 101, an all-digital audio and tone signal synthesis generator for the right ear 102, and a control module 20.

Said all-digital audio and tone signal synthesis generator produces audio signals required for tinnitus diagnosis. Said audio signals are comprised of the signals of pure tone, noise, composite sound, et al. Each ear (the left ear and the right ear) is equipped with an all-digital audio and tone signal synthesis generator, having an adjustable range of acoustic pressure level of 0db-120db in broadband range (such as 30 KHz). Said all-digital audio and tone signal synthesis generator is capable of generating various sound digital signals from mono to compound tones, such as pure tone and/or noise and/or composite sounds and/or simulated animal sounds and/or simulated human languages, and adjusting frequency and/or loudness and/or tone and/or melody and/or rhythm freely.

As shown in FIG.2, said all-digital audio and tone signal synthesis generator includes a pure tone signal generating module 11, a noise signal generating module 12, a composite sound signal generating module 13, a recording signal generating module 14, an audio signal calibration module 15 and an audio signal outputting module 16.

Said pure tone signal generating module 11 generates pure tone audio signals. Pure tone is an audio signal with a single frequency and intensity, which could be single continuous or discontinuous.

Said noise signal generating module 12 generates noise audio signals. The noise is an audio signal irregularly composed of audio signals with different frequency and intensity, and the noise could be single continuous or discontinuous. The noise includes white noise, colored noise transformed through a filter, and noise with adjustable bandwidth.

Said composite sound signal generating module 13 generates audio and tone signals of composite sounds according to specific synthesis rules. Said composite sound signal generating module includes a multi-track synthesis module 131, a signal waveform presetting module 132, and a tinnitus signal library module 133.

There are mainly three ways of forming composite sounds:
The first way involves multi-track synthesis, which comprises one or more separate single track signal generator 1311. The 128 standard tones and the 81 percussion instruments arrangement generated by the digital music / electronic musical instruments international standard "electronic musical instrument digital interface MIDI" are used, the logic algorithm and parameters are changed into digital signals to be input into the digital signal processor DSP or processed by the central processing unit CPU of PC to make a sound. Each single track signal generating could make a sound at any given ranges of frequency and acoustic pressure level, for example, 10 Hz-30 KHz and 0 dB-120 dB. Multiple independent audio signals (such as generated by two or more single track signal generator) are mixed and synthesis quantitatively and qualitatively according to predetermined synthesis manner, like an orchestra according to the score to play a music symphony, generating the composite sound of desired tone, melody, rhythm and loudness. Each sound track is just like a single instrument, of which the audio signal variables include frequency and loudness. Each sound track's contribution to the composite sound mainly depends on the arrangement and loudness at the given frequency. The tone, melody, rhythm and loudness of composite sound audio and tone signal depend on the arrangement of multiple mono-track signal generators and the contribution of multiple mono-track signal generators. The overall loudness of composite sound can be adjusted individually.

The second way involves presetting waveform. Parameters can be adjusted according to the preset audio signal waveform, such as waveform's slope, waveform's amplitude, the prolongation of waveform's peak value, wave period and waveform duration, etc. The abscissa of waveform indicates the time, the ordinate represents the sound loudness. Normal preset waveform includes straight wave, shock wave, ramp wave, sawtooth wave, trigonometric function wave (sine wave, cosine wave, tangent wave, cotangent wave), trapezoidal wave, rectangular wave, impulse wave, white noise and colored noise, etc.

The third way involves tinnitus sound signal library. The library arranges all kinds of tinnitus digital signals collected after clinical medical verification for classified storage and calling. According to the personal testing demands of tinnitus patients, it can restore and output a tinnitus sound signal meeting the patients' personalizing diagnosis requirements by fine-tuning the called parameters of reference tinnitus sound signals, including but not limited to frequency, loudness, waveform, etc. Tinnitus diagnostic technicians can store the diagnostic results of all patients in clinical tinnitus diagnosis into said tinnitus signal library, in order to facilitate calling for reference or medical classification statistical analysis in the future.

Said recording signal generating module 14 generates recorded audio signals. Recording audio signals means adjusting corresponding parameters including, but not limited to, frequency and loudness, according to the inputted or recorded audio tone signals, so as to output a synthesis sound signal meeting the patient personalizing requirements.

Said audio signal calibration module 15 conducts acoustic pressure level or intensity calibration under a given frequency via a built-in or external hearing analysis meter, for the whole audio hardware and software system including specially configured speakers and headphones. Said audio signal calibration module 15 is capable of performing the acoustic pressure level or intensity calibration automatically or manually within any given ranges of frequency and acoustic pressure, for example, 10Hz-30KHz and 0dB-120dB. A headphone with speakers are placed in a sound chamber simulating the human ears to conduct gas pilot test calibration.

Said audio signal outputting module 16 outputs modulated audio and tone signals.

Said control module controls the all-digital audio and tone signal synthesis generator. It could be site control or remote control.

When the tinnitus patient couldn't be present or tinnitus couldn't happen during the test for those intermittent tinnitus patients, according to the description of tinnitus, it is possible to design several audio and tone prescriptions of tinnitus treatment similar to the patient tinnitus. Patients can download the audio and tone prescription to the tinnitus therapeutic apparatus and wear it at home when tinnitus, then feel and debug those several preset prescriptions to find one or more prescriptions similar to his or her own tinnitus. On the basis of the patient feedback, therapists can assist patients to narrow the experimental range and quickly confirm the tinnitus diagnosis. The tinnitus therapeutic apparatus can be classified into two groups, with or without the hearing compensation function.

The tinnitus signals may be debugged remotely and transferred through open mobile platform. A wireless transmitter module GPRS is installed on a tinnitus therapeutic apparatus to realize the direct communication and signal transmission from machine to machine.

The tinnitus diagnosis could be performed in one ear or both ears simultaneously and alternately. Simultaneous and interactive diagnosis in both ears can optimize and steadily restore tinnitus in both ears and has better effect for most patients. The diagnosis for the left ear and the right ear could be copied from each other, then further adjusted individually. The all-digital audio and tone signal synthesis generator for the left and right ear can play and debug simultaneously and alternately.

Tinnitus treatment prescription mainly includes two physical methods: tinnitus masking and tinnitus retraining, which are based on retraining or recoding the neutral system to reduce the central excitability, increase the central suppression, cut the vicious cycle of tinnitus and bad mood, so that the patients can accommodate tinnitus, do not perceive or reduce the perception of tinnitus, so as to achieve the purpose of treatment. The output audio and tone signal of the tinnitus diagnosis includes pure tone, pulse pure tone, mixed pure tone, mixed pulse pure tone; white noise, colored noise, wideband and narrowband noise; single warble tone, mixed warble tone, pulse warble tone; recording audio signal; preset various tinnitus sound and nature sound module; the analysis and masking of signal data such as Fredman masking curve, masking audiogram, the upper band and the lower band of the masking thresholds, the identification and output of the retraining therapy prescription. Tinnitus treatment prescription is the positioning or the audio and tone digital expression of tinnitus sound signals, comprising composite parameters (including tone, rhythm, melody, etc.) and basic parameters (including central frequency, frequency bandwidth, loudness, etc.). The prescription could be saved as an audio signal diagram in PDF picture file format for printing or storing, or directly output the frequency domain audio in compressed or uncompressed format (such as, .wav, .mp3, .mp4, .mp5, .cad, .ram, .wma, .mid, etc.), through converting the time-domain waveform signal into frequency-domain signal by the encoder. It could be downloaded to a tinnitus therapeutic apparatus based on audio signal player, which would be provided to a patient for wearing and listening after decoding. Furthermore, the prescription also could be seamlessly input into a hearing aid fitting module, after debugged through programing, to fit a personalized hearing aid with hearing compensation and tinnitus therapy for the hearing-impaired patients with tinnitus.

Although particular embodiments of the invention have been described as above, it will be understood that they are not intended to limit the patent scope of the present invention, and it will be obvious that the equivalent structural changes or process transformation based on the description and drawings of the present invention should be covered within the scope of the present invention, as well as the embodiments applied in the other relevant technical field directly or indirectly.

## Claims

1. A tinnitus diagnosing and testing device, comprising:
an all-digital audio and tone signal synthesis generators (101, 102), generating audio and tone signals of all kinds of tones, melodies, rhythms and loudness required for diagnosing a variety of tinnitus mode and/or tinnitus sound;
a control module (20), controlling said all-digital audio and tone signal synthesis generators to comprehensively assess tinnitus and output audio and tone signal of the tinnitus diagnosis,
said all-digital audio and tone signal synthesis generators including a composite sound signal generating module, a pure tone signal generating module, a noise signal generating module and a record signal generating module, said composite sound signal generating module, the pure tone signal generating module, the noise signal generating module and
the record signal generating module generating specific audio and tone signals respectively,
wherein said composite sound signal generating module comprises:
a multi-track synthesis module (131), comprising one or more separate single track signal generator (1311), each single track signal generator making sounds at any setting ranges of frequency and acoustic pressure level of pure tone, noise, impulse, etc., and each single sound track's contribution to the composite sound depending on the loudness at the given frequency, the tone, melody, rhythm and loudness of the audio and tone signal of composite sound depending on the arrangement of multiple mono-track signal generators and the contribution of each mono-track signal generator, the overall loudness of the composite sound being able to adjusted individually;
a preset signal waveform module (132), for adjusting the waveform parameters according to the preset audio signal waveform, including but not limited to straight wave, shock wave, ramp wave, sawtooth wave, trigonometric function wave, trapezoidal wave, rectangular wave and impulse wave; and
a tinnitus signal library module (133), for classified saving and calling all kinds of tinnitus digital signals and background sounds in the nature, according to the personal testing demands of tinnitus patients, restoring and outputting a tinnitus sound signal meeting the patients personalizing diagnosis requirements by fine-tuning and calling the parameters corresponding to tinnitus reference signals, such as frequency, loudness, waveform, etc.

2. A tinnitus diagnosing and testing device according to claim 1 wherein it further comprises:
an all-digital audio and tone signal synthesis generator for the left ear, generating the audio and tone signal required for the left ear;
an all-digital audio and tone signal synthesis generator for the right ear, generating the audio and tone signal required for the right ear;
said control module, controlling said all-digital audio and tone signal synthesis generator for the left ear and said all-digital audio and tone signal synthesis generator for the right ear respectively and/or simultaneously, optimizing the audio and tone signals input into the left and right ears, steadily restoring the tinnitus sounds of the left and right ears,
comprehensive assessing tinnitus and outputting the audio and tone signal of tinnitus diagnosis.

3. A tinnitus diagnosing and testing device according to claim 2 wherein the data of said all-digital audio and tone signal synthesis generator for the left ear and said all-digital audio and tone signal synthesis generator for the right ear can replicate with each other and be adjusted based on the replication.

4. A tinnitus diagnosing and testing device according to claim 4 wherein said parameters of waveform comprises, but not limited to, waveform's slope, waveform's amplitude, the prolongation of waveform's peak value, the prolongation of waveform's valley value, wave period and waveform duration.

5. A tinnitus diagnosing and testing device according to claim 1 wherein the pure tone signals generated by said pure tone signal generating module comprises, but not limited to, normal pure tone, synthesis of pure tone, pulse pure tone and mixed pulse pure tone.

6. A tinnitus diagnosing and testing device according to claim 1 wherein the noises generated by the noise signal generating module comprise white noise, colored noise transformed through a filter, and all kinds of noises with adjustable frequency band-width, tone and loudness.

7. A tinnitus diagnosing and testing device according to claim 1 wherein said all-digital audio and tone signal synthesis generator has an adjustable range of acoustic pressure level of 0db-120db in broadband range.

8. A tinnitus diagnosing and testing device according to claim 1 wherein said tinnitus diagnosing and testing device further comprises a remote control module, which can realize remote control and information transmission.

9. A tinnitus diagnosing and testing device according to claim 1 wherein said tinnitus diagnosing and testing device further comprises an audio signal calibrating module, which can scan and calibrate the audio signal manually or automatically.

## Patentansprüche

1. Tinnitus-Diagnostik- und -Testvorrichtung, umfassend
vollständig digitale Audio- und Tonsignalsynthesegeneratoren (101, 102), die Audio-und Tonsignale aller Arten von Tonsignalen, Melodien, Rhythmen und Lautstärke erzeugen, die zum Diagnostizieren einer Vielzahl von Tinnitusmodi und/oder Tinnitusgeräusche erforderlich sind;
ein Kontrollmodul, das die vollständig digitalen Audio- und Tonsignalsynthesegeneratoren steuert, um umfassend Tinnitus zu beurteilen und Audio- und Tonsignal der Tinnitusdiagnose auszugeben,
wobei die vollständig digitalen Audio- und Tonsignalsynthesegeneratoren ein Kompositklangsignal-Erzeugungsmodul, ein Reintonsignal-Erzeugungsmodul, ein Rauschsignal-Erzeugungsmodul und ein Aufzeichnungssignal-Erzeugungsmodul umfasst, wobei das Kompositklangsignal-Erzeugungsmodul, das Reintonsignal-Erzeugungsmodul, das Rauschsignal-Erzeugungsmodul und das Aufzeichnungssignal-Erzeugungsmodul spezifische Audio- bzw. Tonsignale erzeugen,
wobei das Kompositklangsignal-Erzeugungsmodul umfasst:
ein mehrspuriges Synthesemodul (131), das einen oder mehrere getrennte einspurige Signalgeneratoren (1311) umfasst, wobei jeder einzelne einspurige Signalgenerator Klänge in allen Einstellbereichen der Frequenz und des akustischen Druckpegels von Reinton, Geräusch, Impuls usw. erzeugt und wobei der Beitrag jeder Einzelklangspur des Kompositklangs von der Lautstärke bei der gegebenen Frequenz, dem Ton, der Melodie, dem Rhythmus und der Lautstärke des Audio- und Tonsignals des Kompositklangs je nach der Anordnung der mehreren einspurigen Signalgeneratoren und dem Beitrag jedes einspurigen Signalgenerators abhängt, wobei die Gesamtlautheit des Kompositklangs individuell eingestellt werden kann;
ein Voreinstellungssignalwellenformmodul (132), zum Einstellen der Wellenformparameter gemäß der voreingestellten Audiosignalwellenform, einschließlich, ohne darauf beschränkt zu sein, gerader Welle, Schockwelle, Rampenwelle, Sägezahnwelle, Trigonometriefunktionswelle, Trapezwelle, Rechteckwelle und Impulswelle; und
ein Tinnitussignal-Bibliotheksmodul (133), zum geordneten Speichern und Abrufen aller Arten von digitalen Tinnitussignalen und Hintergrundgeräuschen in der Natur, gemäß den persönlichen Testanforderungen von Tinnituspatienten, Restaurieren und Ausgeben eines Tinnitusklangsignals, das die personalisierenden Diagnoseanforderungen der Patienten durch Feinabstimmung und Abrufen der Parameter erfüllt, die Tinnitusreferenzsignalen entsprechen, wie zum Beispiel Frequenz, Lautheit, Wellenform usw.

2. Tinnitus-Diagnose- und -Testvorrichtung nach Anspruch 1, wobei sie ferner umfasst: einen vollständig digitalen Audio- und Tonsignalsynthesegenerator für das linke Ohr, der das Audio- und Tonsignal erzeugt, das für das linke Ohr benötigt wird;
einen vollständig digitalen Audio- und Tonsignalsynthesegenerator für das rechte Ohr, der das Audio- und Tonsignal erzeugt, das für das rechte Ohr benötigt wird;
wobei das Kontrollmodul den vollständig digitalen Audio- und Tonsignalsynthesegenerator für das like Ohr bzw. den vollständig digitalen Audio- und Tonsignalsynthesegenerator für das rechte Ohr kontrolliert und/oder gleichzeitig die Audio- und Tonsignale optimiert, die in das linke und rechte Ohr eingegeben werden, wobei die Tinnitusgeräusche des linken und rechten Ohrs stetig zurückgeführt werden und der Tinnitus umfassend beurteilt wird und das Audio- und Tonsignal der Tinnitusdiagnose ausgegeben wird.

3. Tinnitus-Diagnose- und Testvorrichtung nach Anspruch 2, wobei die Daten des vollständig digitalen Audio- und Tonsignalsynthesegenerators für das linke Ohr und des vollständig digitalen Audio- und Tonsignalsynthesegenerators für das rechte Ohr sich miteinander replizieren und auf der Basis der Reproduktion eingestellt werden können.

4. Tinnitus-Diagnose- und Testvorrichtung nach Anspruch 4, wobei die Parameter der Wellenform den Anstieg der Wellenform, die Amplitude der Wellenform, die Ausdehnung des Peakwertes der Wellenform, die Ausdehnung des Talwertes der Wellenform, die Wellenperiode und die Dauer der Wellenform umfasst, ohne darauf beschränkt zu sein.

5. Tinnitus-Diagnose- und Testvorrichtung nach Anspruch 1, wobei die Reintonsignale, die vom Reintonsignal-Erzeugungsmodul erzeugt werden, normalen Reinton, die Synthese von Reinton, reiner Impulston und gemischter reiner Impulston umfasst, ohne darauf beschränkt zu sein.

6. Tinnitus-Diagnose- und Testvorrichtung nach Anspruch 1, wobei die Geräusche, die vom Rauschsignal-Erzeugungsmodul erzeugt werden, weißes Rauschen, farbiges Rauschen, das durch ein Filter transformiert ist, und alle Arten von Geräuschen mit einstellbarer Frequenzbandbreite, Ton und Lautheit umfassen.

7. Tinnitus-Diagnose- und Testvorrichtung nach Anspruch 1, wobei der vollständig digitale Audio- und Tonsignalsynthesegenerator einen einstellbaren Bereich des akustischen Druckpegels von 0 dB - 120 dB in einem Breitbandspektrum hat.

8. Tinnitus-Diagnose- und Testvorrichtung nach Anspruch 1, wobei die Tinnitus-Diagnose- und Testvorrichtung ferner ein Fernsteuerungsmodul umfasst, das die Fernsteuerung und Informationsübertragung realisieren kann.

9. Tinnitus-Diagnose- und Testvorrichtung nach Anspruch 1, wobei die Tinnitus-Diagnose- und Testvorrichtung ferner ein Audiosignal-Kalibriermodul umfasst, das das Audiosignal manuell oder automatisch scannen und kalibrieren kann.

## Revendications

1. Dispositif de test et de diagnostic d'acouphènes comprenant :
des générateurs (101, 102) de synthèse de signaux audio et tonaux entièrement numériques, générant des signaux audio et tonaux de tous types de tons, mélodies, rythmes et volumes sonores requis pour diagnostiquer une variété de mode d'acouphènes et/ou de son d'acouphènes ;
un module de commande, commandant lesdits générateurs de synthèse de signaux audio et tonaux entièrement numériques pour évaluer de façon complète des acouphènes, et un signal audio et tonal en sortie du diagnostic d'acouphènes,
lesdits générateurs de synthèse de signaux audio et tonaux entièrement numériques comprenant un module de génération de signal sonore composite, un module de générateur de signal tonal pur, un module de génération de signal de bruit et un module de génération de signal d'enregistrement, ledit module de génération de signal sonore composite, le module de générateur de signal tonal pur, le module de génération de signal de bruit et le module de génération de signal d'enregistrement générant des signaux audio et tonaux spécifiques respectivement,
dans lequel ledit module de génération de signal sonore composite comprend :
un module de synthèse multi-pistes (131) comprenant un ou plusieurs générateur(s) (1311) de piste unique séparée, chaque générateur de signal de piste unique produisant des sons à n'importe quelles plages de réglage de niveau de pression acoustique et de fréquence de ton, bruit, impulsion pur(e) etc., et chaque contribution de la piste sonore unique au son composite dépendant du volume sonore à la fréquence, au ton, à la mélodie, au rythme et au volume sonore donné(e) du signal audio et tonal de son composite dépendant de l'agencement de générateurs de signal mono-piste multiples et de la contribution de chaque générateur de signal mono-piste, le volume sonore général du son composite étant en mesure d'être ajusté individuellement ;
un module de forme d'onde de signal pré-réglée (132) pour ajuster les paramètres de forme d'onde, selon la forme d'onde du signal audio pré-réglée, incluant mais sans s'y limiter l'onde droite, l'onde de choc, l'onde en toit d'usine, l'onde en dents de scie, l'onde à fonction trigonométrique, l'onde trapézoïdale, l'onde rectangulaire et l'onde d'impulsion ; et
un module de bibliothèque de signaux d'acouphènes (133) pour l'enregistrement et le lancement classé de tous les types de signaux numériques d'acouphènes et bruits de fond dans la nature, selon les demandes personnelles de test des patients souffrant d'acouphènes, la restauration et la sortie d'un signal sonore d'acouphène correspondant aux exigences de diagnostic personnalisé des patients par un réglage fin et un lancement des paramètres correspondant aux signaux de référence d'acouphènes comme la fréquence, le volume sonore, la forme d'onde, etc.

2. Dispositif de test et de diagnostic d'acouphènes selon la revendication 1, dans lequel il comprend en outre :
un générateur de synthèse de signaux audio et tonaux entièrement numériques pour l'oreille gauche, générant le signal audio et tonal requis pour l'oreille gauche ;
un générateur de synthèse de signaux audio et tonaux entièrement numériques pour l'oreille droite, générant le signal audio et tonal requis pour l'oreille droite ;
ledit module de commande, commandant ledit générateur de synthèse de signaux audio et tonaux entièrement numériques pour l'oreille gauche et ledit générateur de synthèse de signaux audio et tonaux entièrement numériques pour l'oreille droite, respectivement et/ou simultanément, optimisant l'entrée des signaux audio et tonaux dans les oreilles gauche et droite, restaurant régulièrement les sons d'acouphènes des oreilles gauche et droite, évaluant de façon complète les acouphènes et émettant en sortie le signal audio et tonal du diagnostic d'acouphènes.

3. Dispositif de test et de diagnostic d'acouphènes selon la revendication 2, dans lequel les données dudit générateur de synthèse de signaux audio et tonaux entièrement numériques pour l'oreille gauche et dudit générateur de synthèse de signaux audio et tonaux entièrement numériques pour l'oreille droite peuvent répliquer les unes avec les autres et être ajustées en se basant sur la réplication.

4. Dispositif de test et de diagnostic d'acouphènes selon la revendication 4, dans lequel lesdits paramètres de forme d'onde comprennent, mais sans s'y limiter, la pente de la forme d'onde, l'amplitude de la forme d'onde, la prolongation de la valeur de crête de la forme d'onde, la prolongation de la valeur du creux de la forme d'onde, la période de l'onde et la durée de la forme d'onde.

5. Dispositif de test et de diagnostic d'acouphènes selon la revendication 1, dans lequel les signaux tonaux purs générés par ledit module de génération de signal tonal pur comprennent, mais sans s'y limiter, le ton pur normal, la synthèse de ton pur, le ton pur d'impulsion et le ton pur d'impulsion mélangé.

6. Dispositif de test et de diagnostic d'acouphènes selon la revendication 1, dans lequel les bruits générés par le module de génération de signal de bruit comprennent le bruit blanc, le bruit coloré transformé au travers d'un filtre, et tous les types de bruits avec une largeur de bande de fréquence, un ton et un volume sonore ajustable.

7. Dispositif de test et de diagnostic d'acouphènes selon la revendication 1, dans lequel ledit générateur de synthèse de signaux audio et tonaux entièrement numériques présente une plage ajustable de niveau de pression acoustique de 0db-120db dans la plage de bande large.

8. Dispositif de test et de diagnostic d'acouphènes selon la revendication 1, dans lequel ledit dispositif de test et de diagnostic d'acouphènes comprend en outre un module de commande à distance qui peut réaliser la commande à distance et la transmission d'informations.

9. Dispositif de test et de diagnostic d'acouphènes selon la revendication 1, dans lequel ledit dispositif de test et de diagnostic d'acouphènes comprend en outre un module d'étalonnage de signal audio qui peut scanner et étalonner le signal audio manuellement ou automatiquement.
